# EUROPEAN PATENT SPECIFICATION

(11) **EP 2 903 549 B1**
(45) Date of publication and mention of the grant of the patent: **15.02.2017**
(21) Application number: 13750797.6
(22) Date of filing: 06.08.2013
(51) Int. Cl.: A61B 90/14, A61M 16/04

(54) **HEAD STABILIZER FOR MEDICAL DEVICE(S) INCLUDING AN ENDOTRACHEAL TUBE**
KOPFSTABILISATOR FÜR MEDIZINISCHE VORRICHTUNG(EN) MIT EINEM ENDOTRACHEALTUBUS
STABILISATEUR DE TÊTE POUR DISPOSITIF(S) MÉDICAL(UX) COMPRENANT UN TUBE ENDOTRACHÉAL

(30) Priority: 06.08.2012 US 201261679998 P
(43) Date of publication of application: 12.08.2015
(73) Proprietor: Prometheus Medical Innovations LLC, Waterford, Pennsylvania 16441 (US)
(72) Inventor: DAWSON, Randy J., Waterford, Pennsylvania 16441 (US)
(74) Representative: den Braber, Gerard Paul
(86) International application number: PCT/US2013/053755
(87) International publication number: WO 2014/025754

(56) References cited:
- EP-A1- 2 111 798
- WO-A1-97/33541
- WO-A2-2004/052267
- US-A- 3 464 411
- US-A- 5 479 921
- US-A1- 2005 217 681
- US-A1- 2006 081 263
- US-A1- 2007 135 770

## Description

### Field of the Invention

The present invention relates to devices for head stabilization, primarily for medical procedures. More specifically, the present invention concerns a head stabilizer that may be employed to assist with endotracheal intubations and bronchoscopy procedures (among others) that are performed by one or more persons.

### Description of the Related Art

Traditionally, certain medical procedures, such as bronchoscopy procedures, have been done nasally using conscious sedation. The trend now calls for the patient to be paralyzed and intubated so that the procedure may be performed in an operating room setting.

Some medical facilities, including rural facilities do not have the staff to accommodate physicians performing bronchoscopy procedures. Accordingly, physicians are performing these procedures by themselves.

As should be apparent to those skilled in the art, there are no practical devices available to physicians to assist with such procedures. Specifically, there are no devices available to physicians that help to immobilize a patient's head to facilitate insertion of an endotracheal tube.

U.S. Patent Application Publication No. 2009/0229616 (hereinafter "the '616 Application") describes an apparatus for stabilizing an airway tube. The airway tube holder includes a face plate that is positioned over the patient's mouth. (The '616 Application at paragraph [0041].) The face plate includes an inverted L-shaped channel that is designed to receive an endotracheal tube therein. (The '616 Application at paragraph [0041].) A hand-tightened clamp holds the endotracheal tube in the V-shaped notch formed in the channel (The '616 Application at paragraph [0045].) The face plate is affixed to the patient's head via an adjustable attachment means including an elastic cloth strip. (The '616 Application at paragraph [0052].)

U.S. Patent Application Publication No. 2012/0168571 (hereinafter "the '571 Application") describes an endotracheal tube holder with an elongated body portion having a tube grasping mechanism. (The '571 Application at paragraph [0026].) The body portion includes band openings to accommodate a band of flexible material that extends around the back of the patient's head. (The '571 Application at paragraph [0028].) The tube grasping mechanism includes a rigid plastic material to allow secure attachment of an endotracheal tube. (The '571 Application at paragraph [0029].)

U.S. Patent Application Publication No. 2012/0227747 (hereinafter "the '747 Publication") describes a respiratory facemask that attaches to a person's head via straps or a harness connected via a pair of openings. (The '747 Application at paragraph [0052].) The facemask includes a tube holder for accommodating an endotracheal tube therein. (The '747 Application at paragraph [0057].)

U.S. Patent No. 5,305,742 (hereinafter "the '742 Patent") describes an endotracheal tube holder that is held in place above a patient's mouth via straps . (The '742 Patent at col. 2, line 64, through col. 3, line 7.) The endotracheal tube holder includes an adjustable clamp member that permits clamping onto an endotracheal tube. (The '742 Patent at col. 3, lines 8-18.)

U.S. Patent No. 5,479,921 (hereinafter "the '921 Patent") describes an endotracheal tube stabilizer that includes a head frame, an endotracheal tube support bar, and a ventilator tubing securing clamp. (The '921 Patent at col. 5, lines 7-20.) The head frame includes right and left side projections between which the support bar extends. (The '921 Patent at col. 5, lines 44-48, and at col. 5, lines 62-66.)

U.S. Patent No. 7,360,264 (hereinafter "the '264 Patent") describes a patient immobilization device that includes a backboard with a head end including a pair of paddles that support the head and neck of a patient. (The '264 Patent at col. 3, lines 22-43.) The paddles lie flat with the backboard when not in use. (The '264 Patent at col. 3, lines 44-51.)

U.S. Patent No. 8,001,969 (hereinafter "the '969 Patent") describes an airway stabilization system and method. The airway stabilization system includes a faceplate that is connected to a stabilization collar via straps. (The '969 Patent at col. 7, lines 38-42, and at col. 7, lines 63-67.)

While the prior art provides examples of different devices for stabilizing a person's head for a procedure, such as an endotracheal intubation, the prior art fails to provide a convenient way to stabilize a person's head and, thereby, to facilitate the medical procedure.

### Summary of the Invention

The present invention addresses one or more of the deficiencies identified with respect to the prior art. In one aspect, the invention provides an apparatus as defined in claim 1, which is appended to the description. Other, dependent claims define additional features that a specific embodiment of the invention may comprise and that may provide at least one further advantage.

In one contemplated embodiment, the present invention provides an apparatus for immobilizing a patient's head and securing a medical device. The apparatus includes a bottom plate, a first side plate connected to a first side of the bottom plate and extending upwardly from the bottom plate, a second side plate connected to a second side of the bottom plate, opposite to the first side, extending upwardly from the bottom plate, a first arm extending from the first plate toward the second plate, a second arm extending from the second plate toward the first plate, a connector plate connected between the first arm and the second arms, and an attachment provided on the connector plate, wherein the attachment secures a first portion of the medical device thereto.

In another embodiment, the present invention contemplates that the bottom plate, the first side plate, and the second side plate are substantially planar.

Alternatively, the bottom plate, the first side plate, and the second side plate are integrally formed.

It is contemplated that the apparatus of the present invention may include a first tab along a bottom edge of the first side plate, a first slot along a first side edge of the bottom plate to accommodate the first tab therein, a second tab along a bottom edge of the second side plate, a second tab along a second side edge of the bottom plate to accommodate the second tab therein, the second side edge being opposite to the first side edge, a first clamp disposed on the bottom plate adjacent to the first side edge to secure the first side plate to the bottom plate, and a second clamp disposed on the bottom plate adjacent to the second side edge to secure the second side plate to the bottom plate.

If so, each clamp may include a retaining portion, and a lever attached to the retaining portion. Actuation of the lever may cause the retaining portion to engage a retaining slot in each side plate.

In another contemplated embodiment, the retaining portion may be a semi-circular plate pivotally mounted to the bottom plate and the lever is attached to the semi-circular plate such that rotation of the lever causes the semi-circular plate to engage the retaining slot.

The present invention also contemplates that the apparatus includes a first locking bracket attached to the first side plate on an exterior surface thereof, wherein the first locking bracket defines a first channel between the first locking bracket and the first side plate adapted to receive a first locking portion of the first arm therein, a second locking bracket attached to the second side plate on an exterior surface thereof, wherein the second locking bracket defines a second channel between the second locking bracket and the second side plate adapted to receive a second locking portion of the second arm therein, a first lever disposed on the first locking bracket to secure the first locking portion, and a second lever disposed on the second locking bracket to secure the second locking portion.

In a further contemplated embodiment, the apparatus may include a first set of locking teeth disposed on the first locking portion, a first set of side teeth disposed on the first side plate in an orientation providing an interference fit with the first set of locking teeth, a second set of locking teeth disposed on the second locking portion, and a second set of side teeth disposed on the second side plate in an orientation providing an interference fit with the second set of locking teeth.

In another embodiment, it is contemplated that the first lever is L-shaped, defining a first gripping end that presses the first set of locking teeth into engagement with the first set of side teeth, and the second lever is L-shaped, defining a second gripping end that presses the second set of locking teeth into engagement with the second set of side teeth.

The present invention also provides for an apparatus where the connector plate includes a slot for receiving a portion of the medical device therein, and a lock for securing the portion of the medical device in the slot.

The apparatus of the present invention may include an adapter attachable to the medical device, the adapter being constructed for engagement with the lock.

In a further contemplated embodiment, the adapter may include a cylindrical wall with two lateral extensions extending outwardly therefrom, the lock may be a circular void in the connector plate with a diameter greater than a width of the slot, and rotation of the adapter after insertion into the slot may cause the lateral extensions to become disposed in the circular void, thereby preventing dislodgement of the adapter from the connector plate.

Still further, the apparatus of the present invention may have a flexible arm, and a clamp disposed on the flexible arm. The clamp may be adapted to releasably engage a second portion of the medical device, different from the first portion, thereby providing additional stability for the medical device.

In another contemplated embodiment, the first arm may include a first upper arm portion and a first lower arm portion, the first upper arm portion being pivotally adjustable with respect to the first lower arm portion, and the second arm may include a second upper arm portion and a second lower arm portion, the second upper arm portion being pivotally adjustable with respect to the second lower arm portion.

It is contemplated that the medical device may be an endotracheal tube.

This device uses several separate devices to immobilize not only the patient's head, but also to immobilize the endotracheal tube in relation to the patient's head. This stabilization allows the physician to place more effort into the procedure itself, and to be less concerned with holding the endotracheal tube in place. This also adds an element of safety for the patient because it does not allow the tube to move and reduces the risk of possible trauma due to movement.

### Brief Description of the Drawings

The present invention will now be described in connection with the drawings provided herewith, in which:
Fig. 1 is a top view of a first embodiment of a head stabilizer according to the present invention;
Fig. 2 is a bottom view of the head stabilizer illustrated in Fig. 1;
Fig. 3 is a left side view of the head stabilizer illustrated in Fig. 1;
Fig. 4 is a right side view of the head stabilizer illustrated in Fig. 1;
Fig. 5 is a front view of the head stabilizer illustrated in Fig. 1;
Fig. 6 is a rear view of the head stabilizer illustrated in Fig. 1;
Fig. 7 is a cross-sectional top view of the head stabilizer illustrated in Fig. 1, with the cross-section being taken along the line 7 - 7 as shown in Fig. 6;
Fig. 8 is a perspective view of the head cradle portion of the head stabilizer illustrates in Fig. 1;
Fig. 9 is a perspective illustration of the base plate portion of the head stabilizer illustrated in Fig. 1;
Fig. 10 is a perspective illustration of half of the connector plate portion of the head stabilizer illustrated in Fig. 1;
Fig. 11 is a perspective illustration of one of the arm portions of the head stabilizer illustrated in Fig. 1;
Fig. 12 is a perspective, top view of the side locking bracket portion of the head stabilizer illustrated in Fig. 1;
Fig. 13 is a perspective, bottom view of the side locking bracket illustrated in Fig. 12;
Fig. 14 is a perspective illustration of the locking lever portion of the head stabilizer illustrated in Fig. 1;
Fig. 15 is a perspective illustration of the ventilator adapter portion of the head stabilizer illustrated in Fig. 1;
Fig. 16 is a cross-sectional side view of the ventilator adapter illustrated in Fig. 15, the cross-section being taken along the line 16 - 16 in Fig. 15;
Fig. 17 is a perspective illustration of a second embodiment of a head stabilizer according to the present invention, the embodiment being shown in an unfolded state;
Fig. 18 is a perspective illustration of the head stabilizer illustrated in Fig. 17, with the embodiment being shown in the assembled condition, ready for use;
Fig. 19 is a perspective illustration of a third embodiment of a head stabilizer according to the present invention, where a clamp is provided;
Fig. 20 is an enlarged, perspective view of the clamp portion of the head stabilizer illustrated in Fig. 19;
Fig. 21 is a front view of a fourth embodiment of a head stabilizer according to the present invention, including pivotally adjustable arms connecting the connector plate to the side plates;
Fig. 22 is a left side view of the head stabilizer illustrated in Fig. 21; and
Fig. 23 is a perspective illustration of one of the arms provided on the head stabilizer illustrated in Fig. 21.

### Detailed Description of Embodiment(s) of the Present Invention

The present invention will now be described in connection with one or more embodiments thereof. The discussion of any one embodiment is not intended to be limiting of the present invention. To the contrary, the discussion of selected embodiments is intended to illustrate the breadth of the present invention. After understanding the details provided with respect to the embodiments described herein, those skilled it the art may appreciate that there are numerous variations and equivalents to the embodiments described herein. Those variations and embodiment are intended to be encompasses by the scope of the present invention as in appended claims.

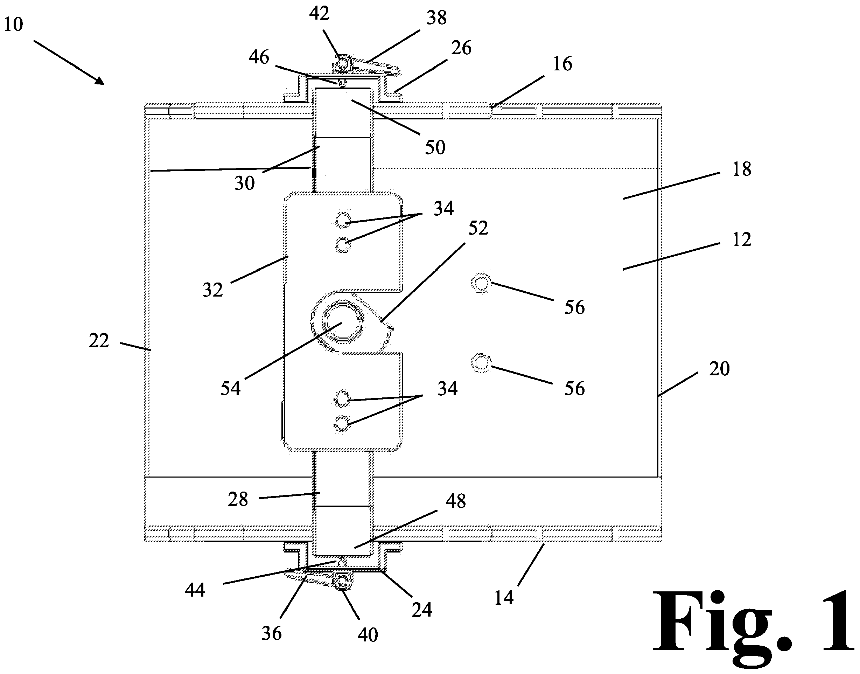

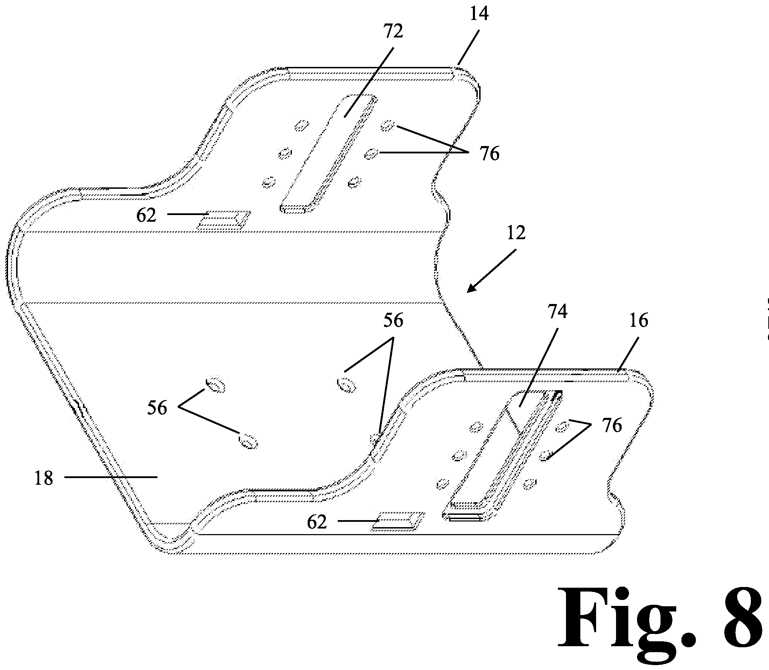

With reference to Fig. 1, a top view of a first embodiment of a head stabilizer 10 is illustrated. The head stabilizer 10 includes a head cradle 12 having a right side 14 and a left side 16. The head cradle 12, which is illustrated in perspective detail in Fig. 8 may be made from any suitable material including plastic, thermoplastic, rubber, nylon, polyurethane, polytetrafluoroethylene, composite materials, metals, or any combination thereof, among others. While a suitable, flexible, light-weight material is contemplated for the construction of the head cradle, the present invention is not contemplated to be limited to any particular material. As should be apparent to those skilled in the art, materials may be selected based upon the preference of the user, among other variables.

The sides 14, 16 are illustrated as being integrally molded to the bottom surface 18 of the head cradle 12. As should be apparent to those skilled in the art, however, this is but one possible construction for the head cradle 12. The sides 14, 16 may be removably connected to the bottom 18. Alternatively, the sides 14, 16 may be manufactured separately from the bottom 18 and connected thereto during assembly of the head cradle 12. Still further constructions are contemplated to fall within the scope of the present invention, as should be apparent to those skilled in the art and as discussed in greater detail in the paragraphs that follow.

With continued reference to Fig. 1, it is noted that a person's head is contemplated to fit between the sides 14, 16 of the head cradle 12. The orientation of the person's head within the head cradle 12 is not particularly relevant to the operation of the present invention. In particular, the top of the person's head may be oriented adjacent to the top edge 20 of the head cradle while the person's neck is oriented adjacent to the bottom edge 22 of the head cradle 12. Of course, as desired by the practitioner, the head cradle 12 may be used in the opposite orientation without departing from the scope of the present invention.

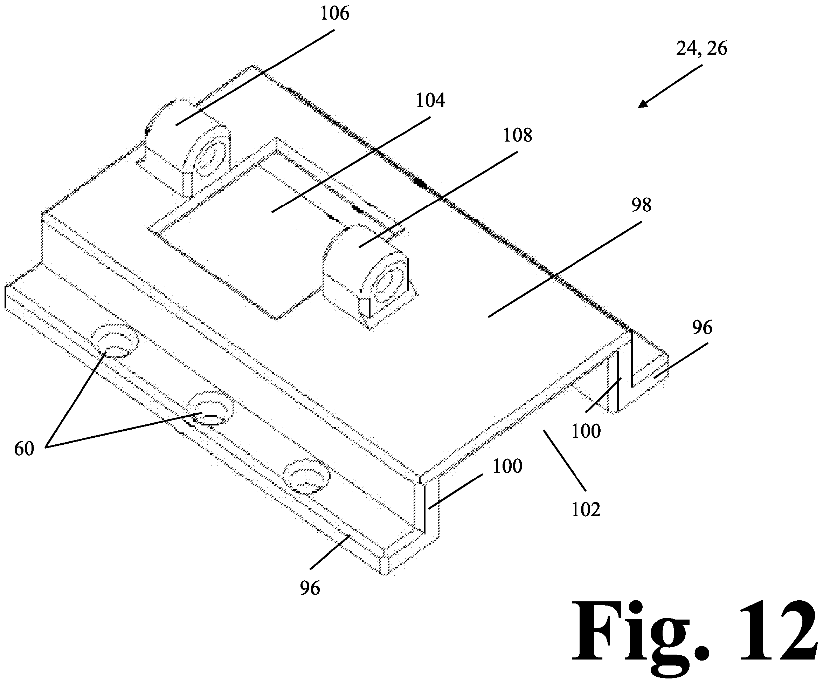

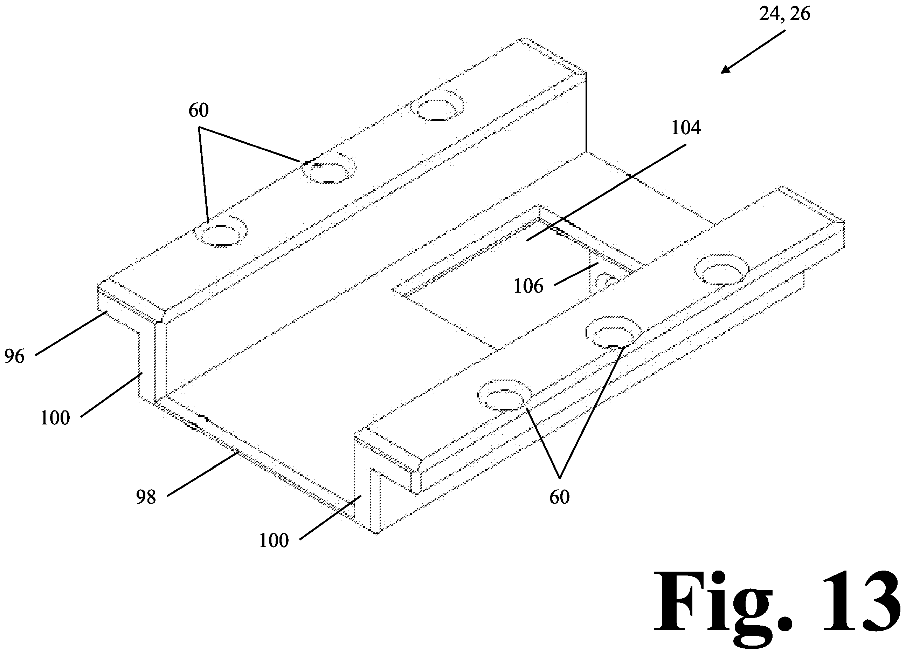

As illustrated in Fig. 1, the head stabilizer 10 includes a left locking bracket 24 and a right locking bracket 26. The left locking bracket 24 is connected to the left side 14 of the head cradle 12. The right locking bracket 26 is connected to the right side 16 of the head cradle 12. Figs. 12 and 13, which are discussed in greater detail below, provide enlarged details concerning the left and right locking brackets 24,26.

It is contemplated that the left and right locking brackets 24, 26 may be made from a suitable material including, but not limited to plastics. As should be apparent, however, the left and right locking brackets 24, 26 may be made from any suitable material without departing from the scope of the present invention. For example, the left and right locking brackets 24, 26 may be made from thermoplastics, rubber, polyurethane, polypropylene, polytetrafluoroethylene, nylon, metal, composite materials, natural materials, and the like. The exact composition of the locking brackets 24, 26 is not intended to be limiting of the present invention.

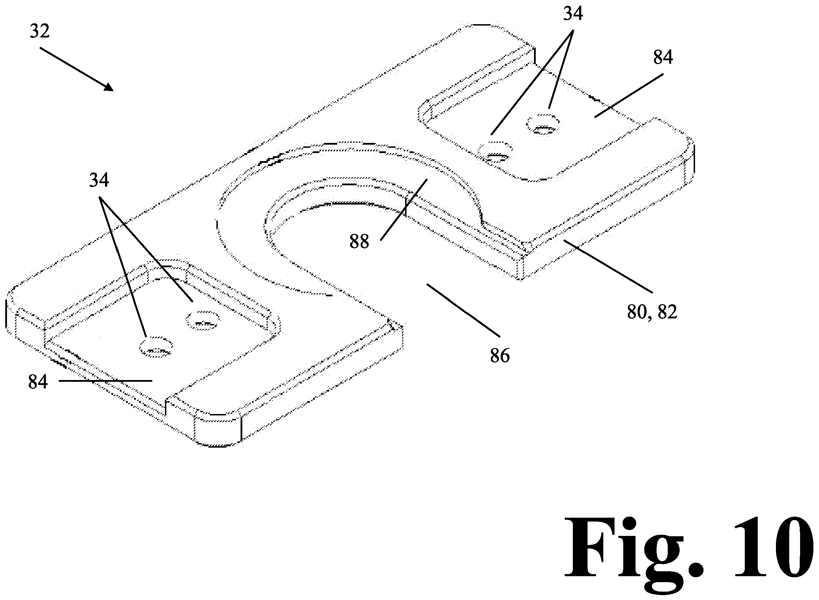

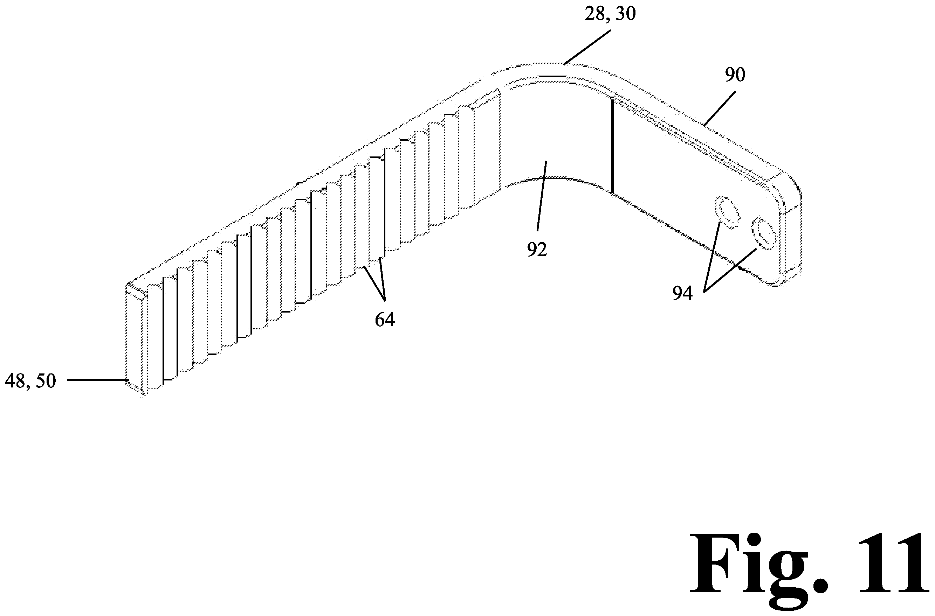

As illustrated in Fig. 1, left and right arms 28, 30 extend upwardly from the left and right locking brackets 24, 26. The left and right arms 28, 30 meet at and are connected to a connector plate 32. Details of the left and right arms 28, 30 (also referred to herein as the left and right locking arms 28, 30) are provided in connection with Fig. 11. Details of the connector plate 32 are provided in connection with Fig. 10.

Because the arms 28, 30 and the connector plate 32 are positioned so that they hold an endotracheal tube securely in a person's throat, the arms 28, 30 and the connector plate 32 are contemplated to be made from materials that are resistant to growth and transmission of microorganisms. Suitable materials include, but are not limited to plastics, thermoplastics, rubber, polyurethane, polypropylene, polytetrafluoroethylene, metal, composite materials, natural materials, and the like. The exact composition of the arms 28, 30 and the connector plate 32 is not critical to the practice of the present invention.

With respect to the arms 28, 30, it is noted that the arms 28, 30 are contemplated to be made from a material with some flexibility. The flexibility of the arms 28, 30 is contemplated to facilitate positioning of the connector plate 32 over a person's mouth. Flexible arms 28, 30 also are contemplated to facilitate placement and securement of the endotracheal tube secured in the connector plate 32.

As shown in Fig. 1, the arms 28, 30 are contemplated to connect to the connector plate 32 via one or more fasteners 34 so that the arms 28, 30 are removably connected thereto. In an alternative arrangement, it is contemplated that the arms 28, 30 may be molded with or permanently affixed to the connector plate 32.

To permit a practitioner to secure the connector plate 32, once positioned over a person's mouth, the head stabilizer 10 includes left and right locking levers 36, 38. The left and right locking levers 36, 38 are essentially L-shaped members that connect, via left and right pivots 40, 42 to the left and right locking brackets 24, 26. The levers 36, 38 each include gripping ends 44, 46 that engage portions of the arms 28, 30 extending into the locking brackets 24, 26. The portions of the arms 28, 30 that are engaged by the gripping ends 44, 46 are referred to as locking portions 48, 50 herein, for clarity.

As should be apparent from Fig. 1, the levers 36, 38 are contemplated to be operable by the practitioner. Specifically, the levers 36, 38 may be opened to permit the insertion of the arms 28, 30 therein. Once the locking portions 48, 50 of the arms 28, 30 are positioned at a suitable location, the practitioner actuates the levers 36, 38 until the gripping ends 44, 46 engage the locking portions 48, 50 of the arms 28, 30. At this point, the arms 28, 30 are secured in their predetermined positions so that the connector plate 32 is properly positioned above the patient's mouth.

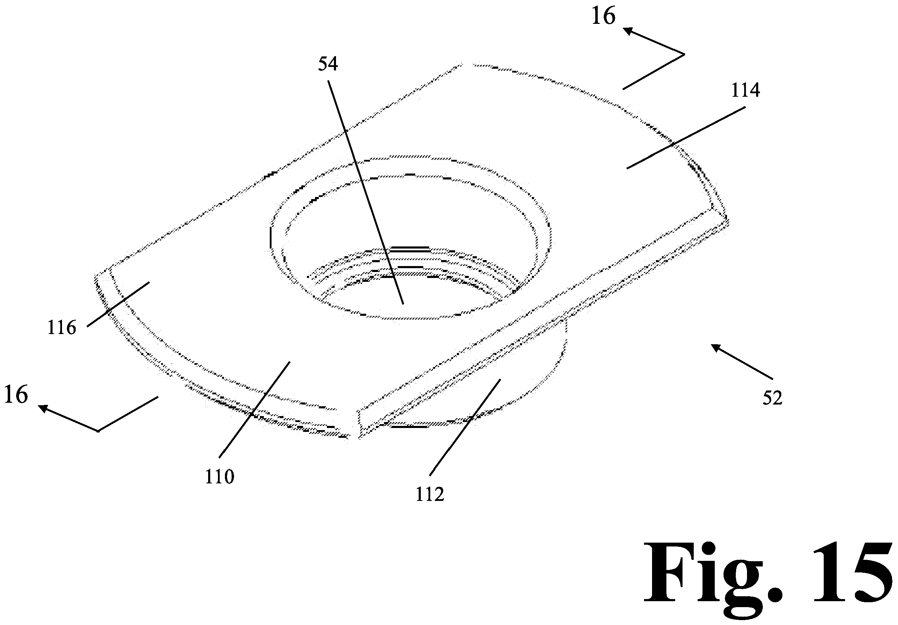

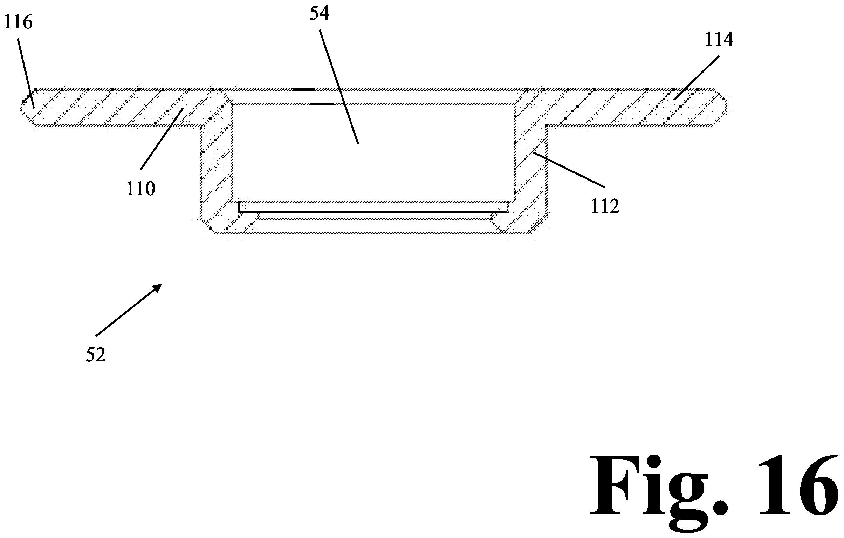

Fig. 1 also illustrates a ventilator adapter 52 that has been positioned in the connector plate 32. The ventilator adapter 52 is illustrated in greater detail in Figs. 15 and 16. The ventilator adapter 52 includes a central opening 54 through which an endotracheal tube, ventilator, bronchoscope, or other suitable medical instrument may be inserted. The ventilator adapter 52 (also referred to herein as "the adapter 52") is contemplated to be removably securable to the connector plate 32.

Fig. 1 also illustrates fastener holes 56 in the bottom 18 of the head cradle 12. The fastener holes 56 are provided so that the head cradle 12 may be affixed to a suitable substrate, such as a stretcher, gurney, base plate, or the like.

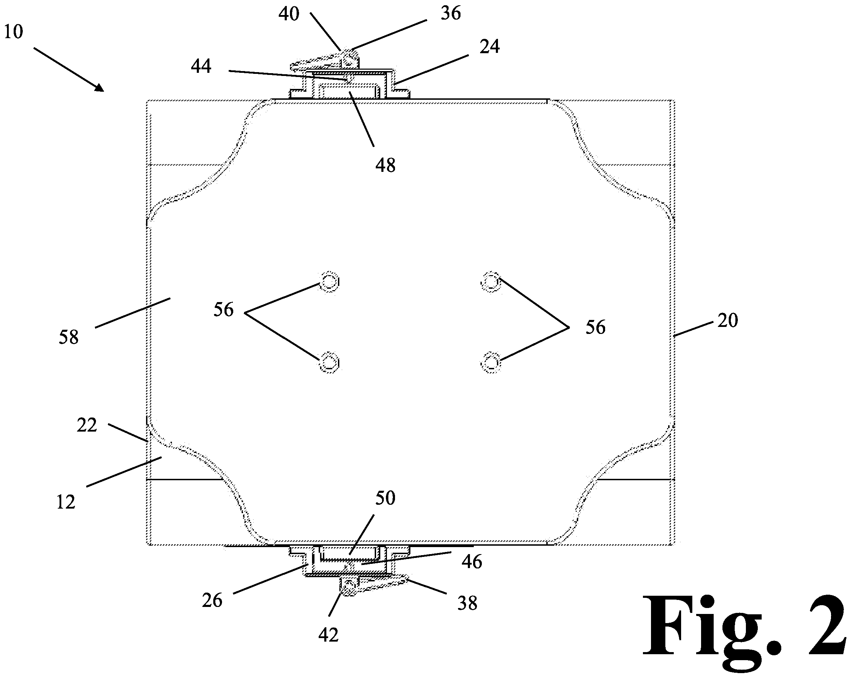

Fig. 2 is a bottom view of the head stabilizer illustrated in Fig. 1. In this view, a base plate 58 has been attached to the bottom of the head cradle 12. The base plate 58 is contemplated to be made from a material that provides a frictional engagement with the surface upon which the head stabilizer 10 is placed. The base plate 58 also may be weighted to provide additional stability for the head stabilizer 10. While the base plate may be made from any suitable material, it is contemplated that the base plate 58 may be made from a dense rubber. In addition, the base plate 58 may include an anti-skid material on the bottom to resist sliding on the table or other surface on which the head stabilizer 10 is placed. The present invention is not contemplated to be limited to any particular material for the construction of the base plate 58 or any anti-skid surface that may be applied thereto.

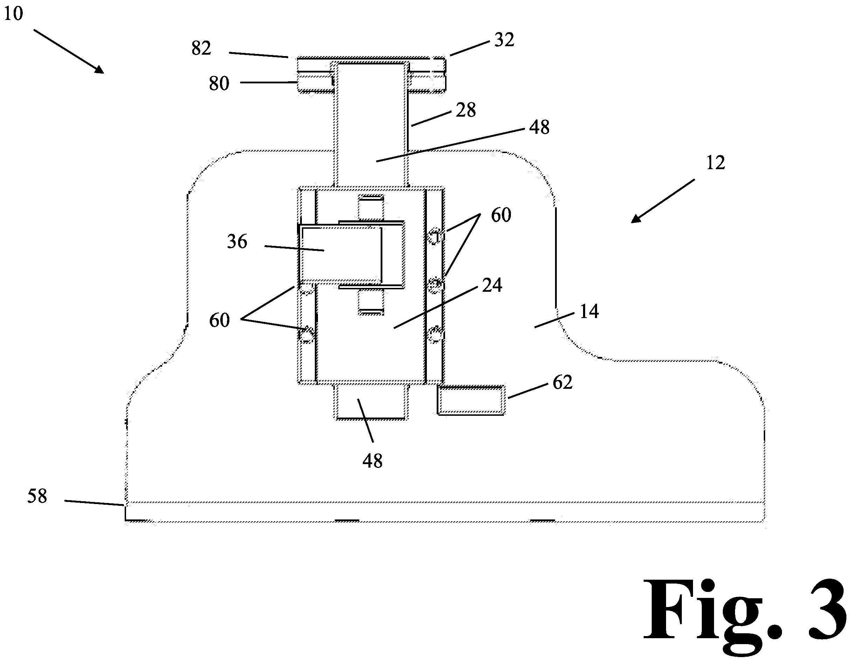

Fig. 3 is a left side view of the head stabilizer 10 of the present invention, which is illustrated in Fig. 1. In this view, fastener holes 60 are visible in the left locking bracket 24. The fastener holes 60 permit the left locking bracket 24 to be removably attached to the left side 14 of the head cradle 12. In an alternative embodiment of the head stabilizer 10 of the present invention, it is contemplated that the left locking bracket 24 is affixed to the left side 14 via a suitable adhesive or welding, among other forms of attachment that should be apparent to those skilled in the art.

Fig. 3 also illustrates one strap opening 62, which may be provided so that one or more straps may be used to secure the head stabilizer 10 to a suitable table or other substrate.

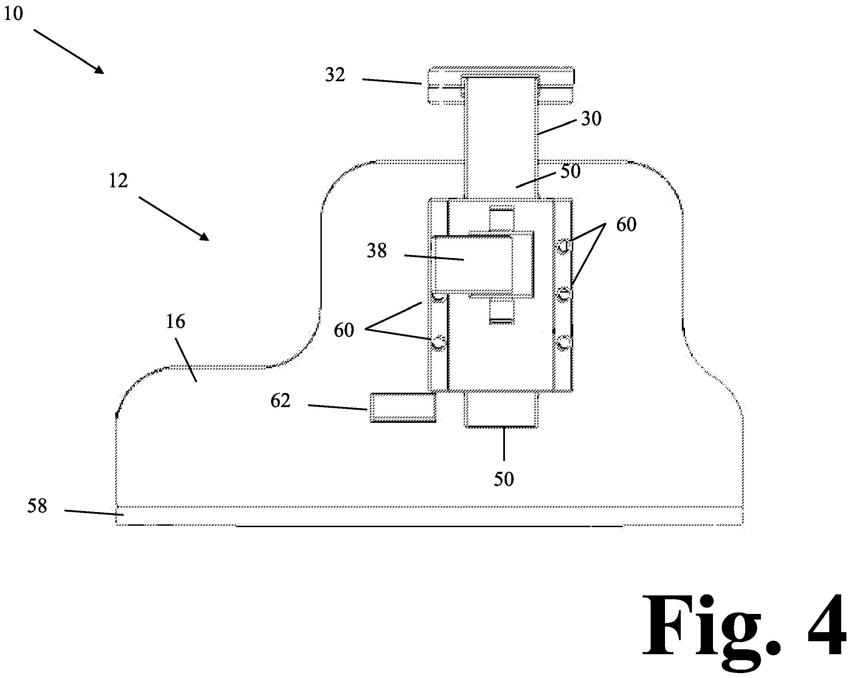

Fig. 4 is a right side view of the head stabilizer 10 of the present invention. As in other figures, like reference numbers are used to refer to like features that are described in connection with Fig. 3.

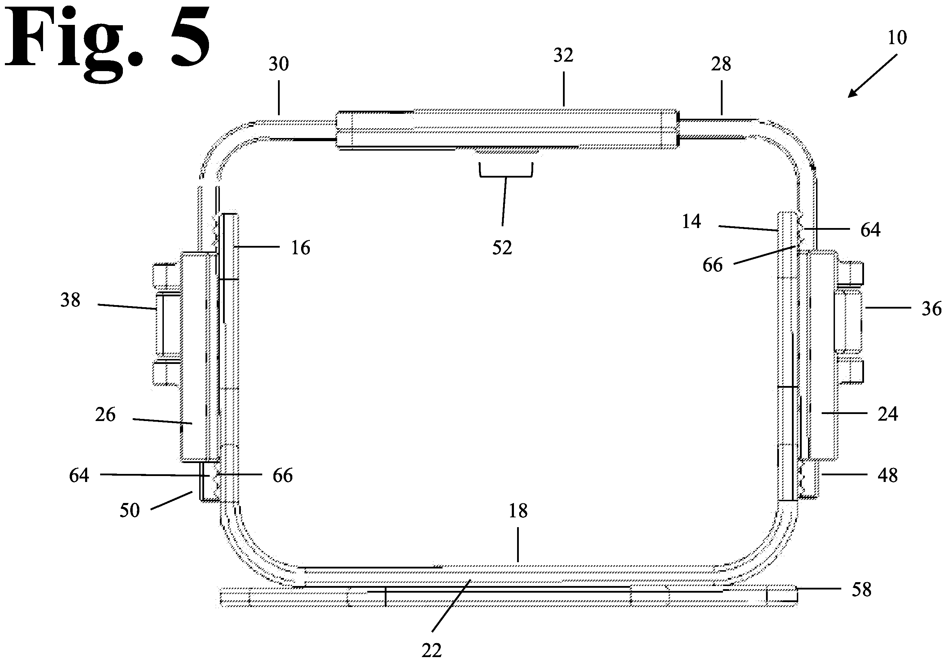

Fig. 5 is a front view of the head stabilizer 10 of the present invention. In this illustration, locking teeth 64 on the locking portions 48, 50 of the arms 28, 30 are visible. Complimentary locking teeth 66 are visible on the left and right sides 14, 16 of the head cradle 12. When the levers 36, 38 are in a locked position, the locking teeth 64, 66 are pressed into engagement with one another, thereby discouraging disengagement of the arms 28, 30 from the sides 14, 16 of the head cradle 12. While the teeth 64, 66 are illustrated as being triangularly-shaped, any other suitable shape is contemplated to fall within the scope of the present invention. In the alternative, teeth 64, 66 need not be employed if the opposing surfaces are otherwise manufactured to provide mutually engaging features (such as a frictional engagement, a tacky engagement, or the like).

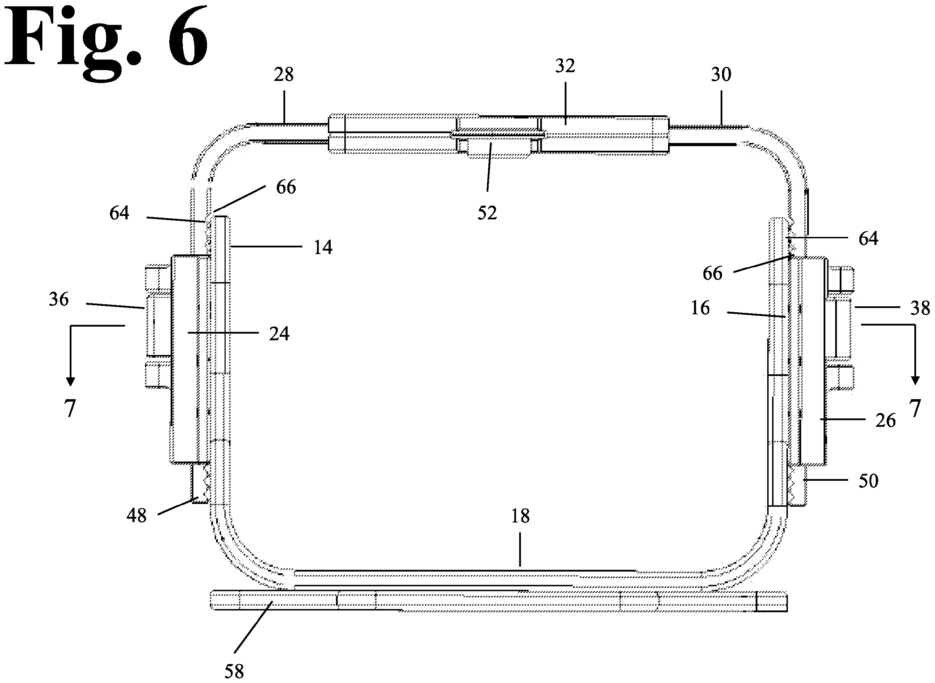

Fig. 6 is a rear view of the head stabilizer 10 of the present invention.

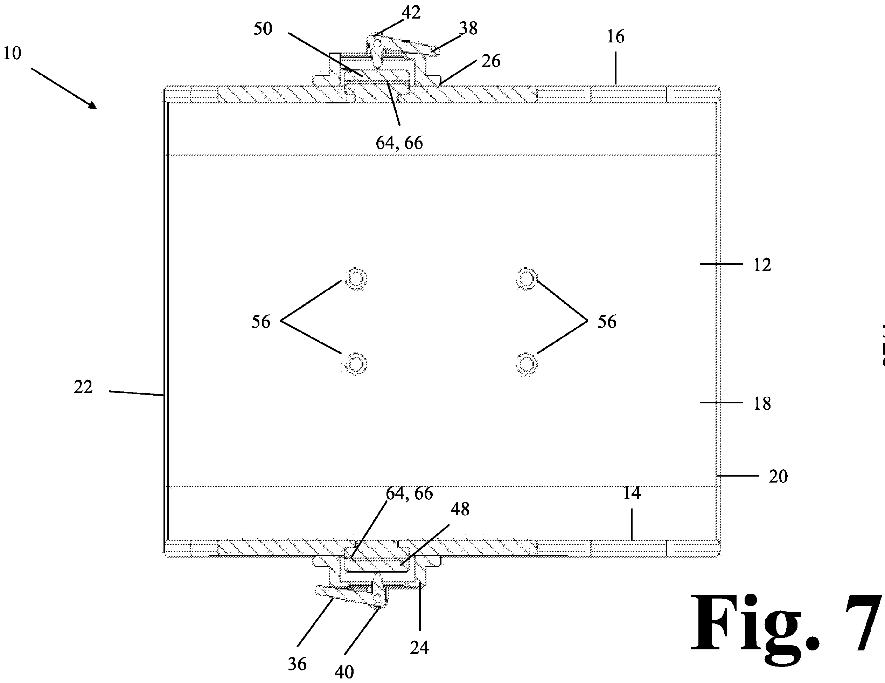

Fig. 7 is a cross-sectional view of the head stabilizer 10 illustrated in Fig. 6, the cross-section being taken along the line 7 - 7 in Fig. 6. In this view, the teeth 66 that are provided on the sides 14, 16 of the head stabilizer 10 are provided on inserts 68, 70 that are integrated into the sides 14, 16 of the head cradle 12. The inserts 68, 70 need not be provided in the case where the sides 14, 16 are molded with the teeth 66 provided on the surface thereof.

Fig. 8 is a perspective illustration of the head cradle 12 portion of the head stabilizer 10 of the present invention. In this view, two openings 72, 74 are provided to accommodate the inserts 68, 70 that include the teeth 66. Fastener holes 76 are provided adjacent to the openings 72, 74 in the sides 14, 16, of the head cradle 12. The fastener holes 76 are contemplated to align with the fastener holes 60 in the locking plates 24, 26 so that the locking plates 24, 26 may be secured to the sides 14, 16.

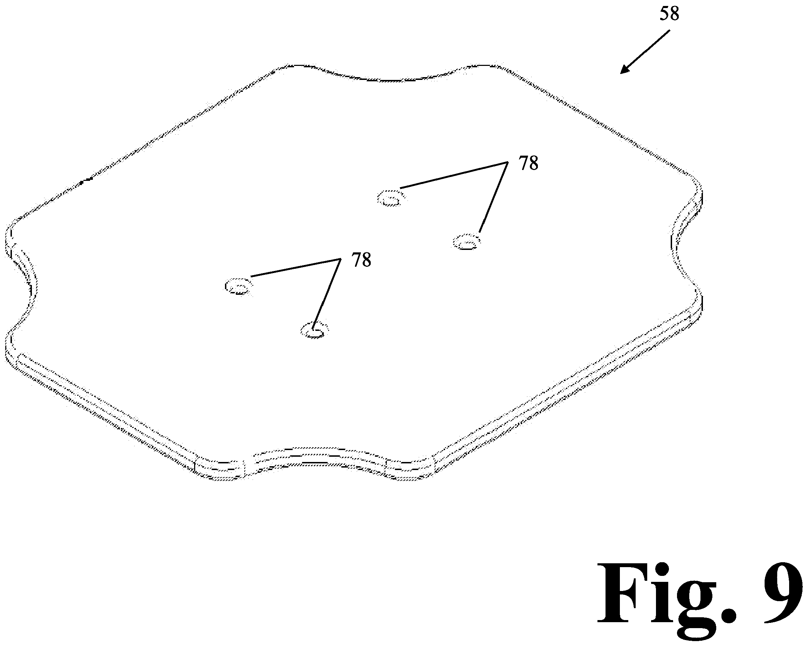

Fig. 9 is a perspective illustration of the base plate 58 that is attachable to the head cradle 12. The base plate includes fastener holes 78 that are provided to align with the fastener holes 56 in the bottom 18 of the head cradle 12.

Fig. 10 is a perspective illustration of a portion of the connector plate 32 that is illustrated in Fig. 1. Specifically, Fig. 10 illustrates a bottom portion 80 of the connector plate 32. The top portion 82 (shown in Fig. 3) is a mirror image of the bottom portion. The top portion 82 and the bottom portion 80 are intended to be assembled to form the connector plate 32, as shown.

The bottom plate 80 and the top plate 82 include fastener holes 34 that, when the plates 80, 82 are stacked on each other, are in register with one another. The plates 80, 82 include indentations 84 at either end that accommodate the arms 28, 30 therein. The plates 80, 82 also include a slot 86 that extends to a circular adapter lock 88. The slot 86 is constructed to permit an endotracheal tube (or other medical instrument) to be inserted therein. The adapter lock 88 is provided so that the ventilator adapter 52 may be locked therein, thereby securing the medical device in the connector plate 32.

In an alternative embodiment, it is contemplated that the connector plate 32 may be manufactured as a unitary component of the head stabilizer 10 of the present invention. Still further constructions for the connector plate 32 are contemplated to fall within the scope of the present invention, as should be apparent to those skilled in the art.

Fig. 11 is a perspective illustration of one of the arms 28, 30 that are part of the head stabilizer 10 of the present invention. The arm 28, 30 includes a top portion 90 connected to the locking portion 48, 50 via a bend 92. The top portion 90 includes two fastener holes 94 that are positioned to be in register with the fastener holes 34 in the connector plate 32. Since the arms 28, 30 are mirror images of one another, the arms 28, 30 are identical except for their respective positions on either side of the connector plate 32.

Fig. 12 is a perspective illustration of one of the locking brackets 24, 26 that are connected to the sides 14, 16 of the head cradle 12. The locking brackets 24, 26 have the same construction in the illustrated embodiment of the head stabilizer 10. As should be apparent, however, the locking brackets 24, 26 may differ from one another without departing from the scope of the present invention.

Each locking bracket 24, 26 includes two side portions 96 and a central portion 98 that is disposed at a different elevation than the side portions 96. Walls 100 connect the side portions 96 to the central portion 98. With this construction, the locking brackets 24, 26, after being attached to the sides 14, 16, establish channels 102 into which the locking portions 48, 50 of the arms 24, 26 may be inserted.

Each locking bracket 24, 26 also includes a central opening 104 through the central portion 98. The central opening accommodates the gripping end 44, 46 of the lever 36, 38 inserted thereinto. Two pivot supports 106, 108 are disposed, one on each side of the central opening 104. The pivot supports 106, 108 receive an axle (or rod) that defines the pivots 40, 42 of the levers 36, 38.

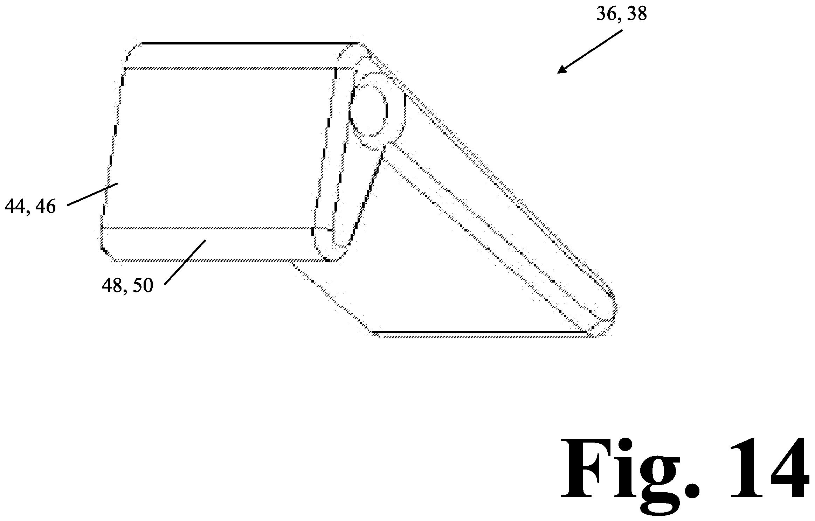

Fig. 14 is a perspective illustration of one of the levers 36, 38 forming a part of the head stabilizer 10 of the present invention.

Fig. 15 is a perspective illustration of the ventilator adapter 52 portion of the head stabilizer 10 of the present invention. The ventilator adapter 52 includes a support plate 110 including a cylindrical wall 112. The cylindrical wall 112 defines the central opening 54 in the ventilator adapter 52. The support plate 110 includes two lateral extensions 114, 116 that extend outwardly from the cylindrical wall 112.

The lateral extensions 114, 116 have curved ends 118, 120 that define a circle. The curved ends 118, 120 are contemplated to fit within the adapter lock 88 defined within the connector plate 32. As illustrated, the width of the lateral extensions 114, 116 is greater than a width of the slot 86. Similarly, the diameter of the adapter lock 88 is greater than the width of the slot 86. As a result, when the lateral extensions 114, 116 are rotated from the initial orientation parallel to the axis of the slot 86, the adapter 52 is retained by the adapter lock 88.

To connect the ventilator adapter 52 to the connector plate 32, the ventilator adapter 52 is inserted into the slot 86 such that the lateral extensions 114, 116 are parallel to the slot 86. After the interior one of the lateral extensions 114, 116 reaches the limit of the slot 86 and adapter lock 88, the ventilator adapter 52 is rotated, as illustrated in Fig. 1. Once rotated, the lateral extensions 114, 116 are no longer in register with the slot 86. The lateral extensions 114, 116 become trapped by the adapter lock 88, which prevents the ventilator adapter 52 from being inadvertently dislodged from the connector plate 32. As noted above, the ventilator adapter 52 receives an endotracheal tube (or other medical device) therein. Thus, when the ventilator adapter 52 is captured by the connector plate 32, the medical device is secured to the head stabilizer 10.

Fig. 16 is a cross-sectional illustration of the ventilator adapter 52 shown in Fig. 15. The illustration provides additional details concerning the ventilator adapter 52.

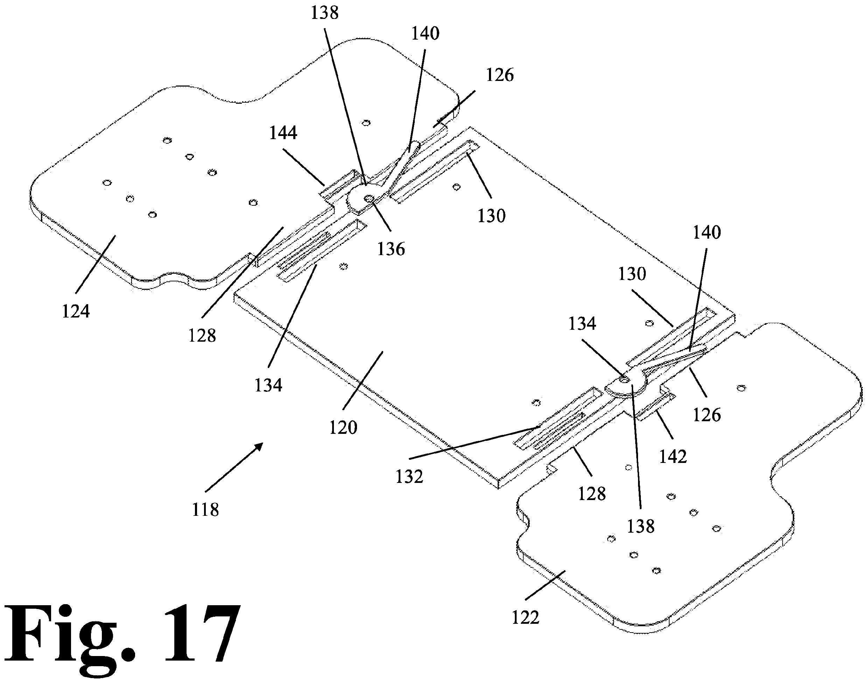

Fig. 17 is a perspective illustration of a second embodiment of a head stabilizer 118 according to the present invention. The head stabilizer 118 includes a bottom portion 120, a left side 122, and a right side 124. The left side 122 and the right side 124 are constructed so that they may be stored in a flat condition and be connected to the bottom portion 120 before use of the head stabilizer 118.

In the illustrated embodiment, each side 122, 124 includes two tabs 126, 128 that are disposed so that they may be inserted into slots 130, 132 in the bottom portion 120 of the head stabilizer 118. Before use the tabs 126, 128 are inserted into the slots 130, 132 so that the sides 122, 124 are vertically oriented with respect to the bottom portion 120.

The bottom portion 120 also includes two retaining clamps 134, 136. The retaining clamps 134, 136 each include a retaining portion 138 and a lever portion 140. The retaining claims 134, 136 are rotationally mounted on the base portion 120. After the sides 122, 124 are positioned such that the tabs 126, 128 are disposed in the slots 130, 132, the lever 140 is rotated so that the retaining portion 138 engages a retaining slot 142, 144 in the associated side 122, 124. In this manner, the sides 122, 124 are removably attached to the bottom portion 120.

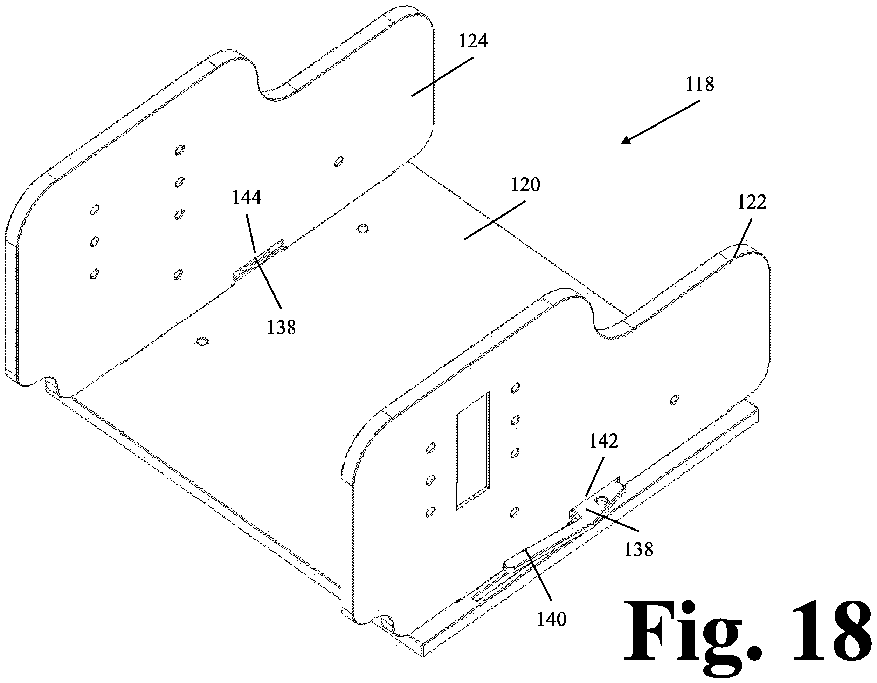

Fig. 18 is a perspective illustration of the head stabilizer 118, after the sides 122, 124 have been attached to the bottom portion 120. The levers 140 have been rotated so that the retaining portions 138 engage their respective slots 142, 144 in the sides 122, 124.

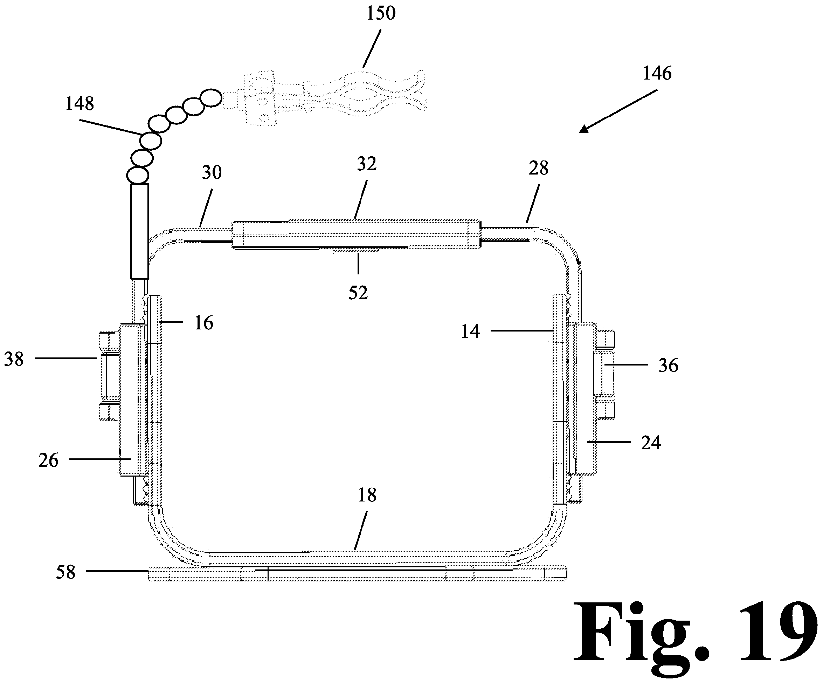

Fig. 19 is a front view of a third contemplated embodiment of the head stabilizer 146 of the present invention. In this embodiment, which is a variation of the embodiment illustrated in Fig. 1, a flexible arm 148 and a clamp 150 have been added. While the flexible arm 148 is shown as being attached to (or incorporated as a part of) one of the arms 30, the flexible arm 148 may be attached at any suitable location on the head stabilizer 118, as required or as desired. The clamp 150 is provided to grab onto any portion of the endotracheal tube that extends above the connector plate 32. The clamp 150, therefore, helps to stabilize the medical device being used in conjunction with the head stabilizer 146.

It is noted that the flexible arm 148 may have any suitable construction. In other words, the flexible arm 148 is not limited to any particular construction in connection with the present invention. In addition, the flexible arm 148 may be made from any suitable material.

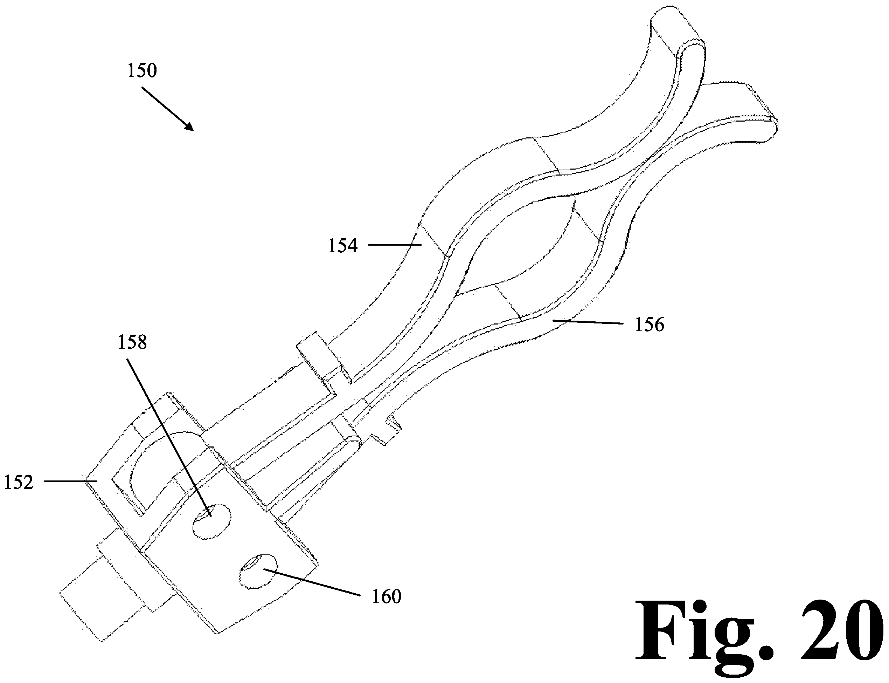

Fig. 20 is a perspective view of the clamp 150 illustrated in connection with the head stabilizer 146 shown in Fig. 19. The clamp 150 includes a base 152 that connects to the flexible arm 148. The base 152 includes two clamp elements 154, 156 that are permitted to pivot around respective pivots 158, 160. It is contemplated that the clamp elements 154, 156 are biased into a closed position. As a result, medical devices may be removably held thereby.

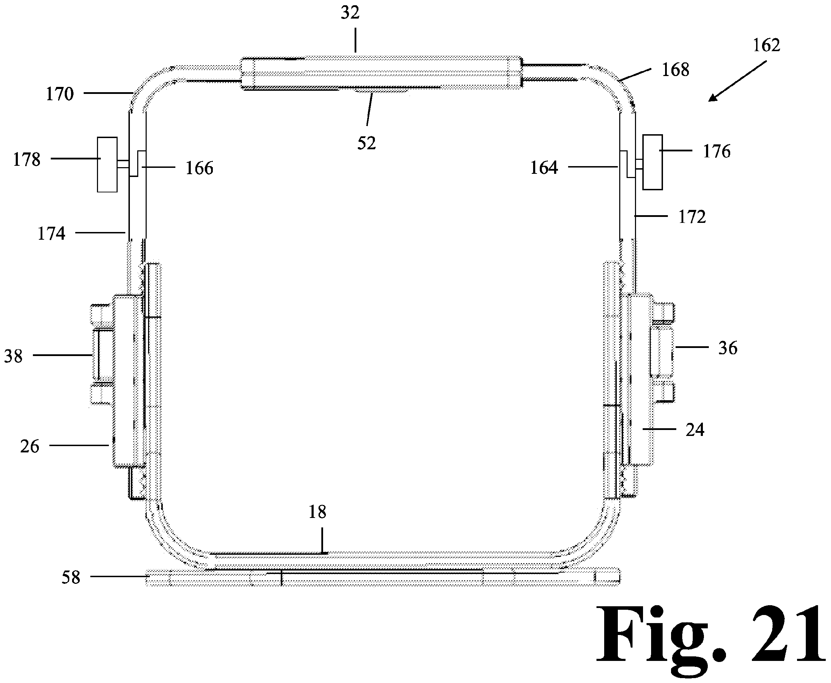

Fig. 21 is a front view of a fourth contemplated embodiment of a head stabilizer 162 according to the present invention. This embodiment is similar to the head stabilizer 10, except that the arms 164, 166 are pivotally adjustable in this fourth embodiment. Specifically, the arms 164, 166 are constructed so that the connector plate 32 may be pivoted toward the top edge 20 or the bottom edge 22 of the head cradle 12. This additional motion provides further flexibility to the head stabilizer 162 to facilitate endotracheal intubation (or other medical procedures) as noted herein.

As shown in Fig. 21, the arms 164, 166 include upper arm portions 168, 170 and lower arm portions 172, 174. The upper arm portions 168, 170 connect pivotally to the lower arm portions 172, 174 and are adjustable with respect to one another via knobs 176, 178. The knobs 176, 178 may be tightened or loosened to permit adjustment of the upper arm portions 168, 170 with respect to the head cradle 12.

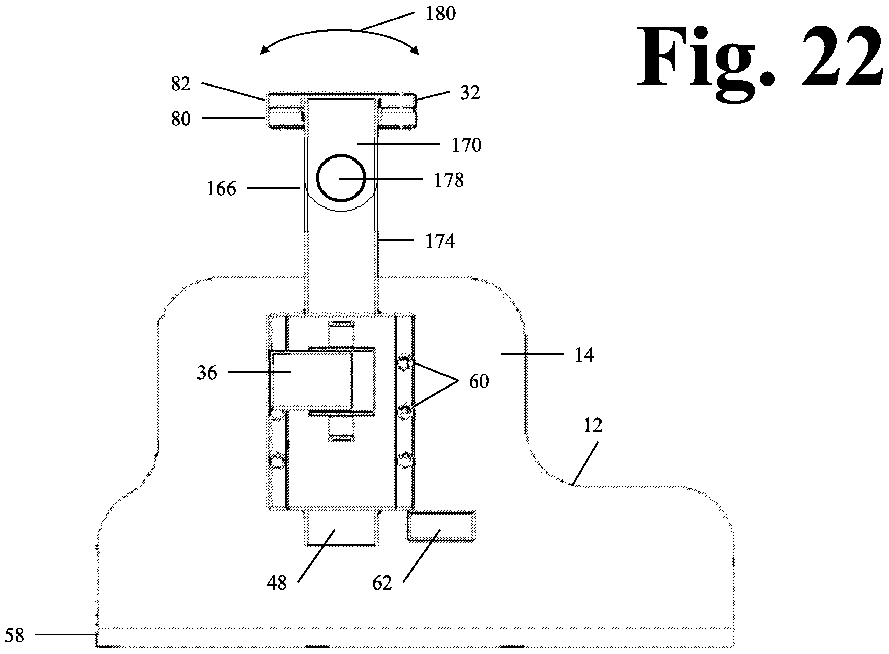

Fig. 22 is a left side view of the head stabilizer 162 illustrated in Fig. 21. Arrows 180 are provided to indicate the direction in which the upper arm portions 168, 170 and connector plate 32 may be pivoted with respect to the head cradle 12.

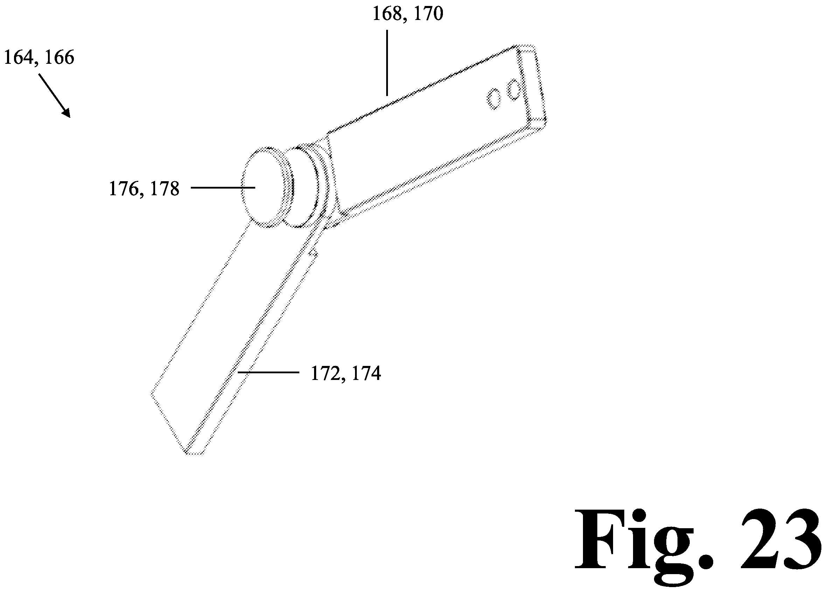

Fig. 23 is a perspective illustration of one of the arms 164, 166, illustrating the upper arm portion 168, 170 tilted with respect to the lower arm portion 172, 174.

In connection with the various embodiments described above, it is noted that the individual components of the head stabilizers 10, 118, 146, 162 may be made from any suitable material including, but not limited to plastic, thermoplastic, rubber, nylon, polyurethane, polytetrafluoroethylene, composite materials, metals, or any combination thereof, among others. In other words, the exact compositions of any individual component is not considered to be limiting of any aspect of the present invention.

Concerning the ventilator adapter 52, it is noted that the ventilator adapter 52 may be constructed to accommodate a standard endotracheal tube or other medical instrument. In one embodiment, the present invention contemplates that the opening 54 will have a diameter of about 15 mm (0.59 in.), which is a standard size. As should be apparent, this diameter is merely exemplary of one possible construction. It is contemplated that any of a large number of adapters 52 may be provided for the head stabilizer 10, 118, 146, 162 of the present invention. Each adapter 52 may include a central opening 54 that is shaped and sized for a specific medical instrument.

To assist with an understanding of the scope of the present invention, a brief discussion of the use of the head stabilizer 10, 118, 146, 162 is now provided.

The head stabilizer 10, 118, 146, 162 is contemplated to include a connector plate 32 with a slot 86 of sufficient width and proportions to accept the medical device, endotracheal tube, ventilation tube, or other apparatus to be secured by the head stabilizer 10, 118, 146, 162. Regardless of the type of device that is used in connection with the head stabilizer 10, 118, 146, 162, the present invention has, as one of its objects, the ability to assist a practitioner to examine, ventilate, and/or intubate a patient with little or no help from other persons. In other words, the head stabilizer 10, 118, 146, 162 of the present invention is contemplated to become a second or third pair of hands for a practitioner working alone. This may occur, for example, in a remote area or in an emergency, among other circumstances.

As should be apparent from the foregoing, the head stabilizer 10, 118, 146, 162 not only holds the patient's head in a particular orientation, but the apparatus provides a connector plate 32 to which one or more medical devices may be connected. As noted, the connector plate 32 holds the medical device in relation to the patient's mouth so that the practitioner may examine, ventilate, or intubate the patient (among other medical procedures).

In use, it is contemplated that the patient may be lying on a flat surface, such as an examining table, gurney, or the like. However, this is not required to practice the present invention, which is applicable to a patient in any orientation.

As noted above, the practitioner may secure the head stabilizer 10, 118, 146, 162 to the table (or other substrate) via tethers or straps that may connect to the head stabilizer 10, 118, 146, 162 via the openings 62 provided therein (among other possible openings that are not illustrated herein).

Once secured, the practitioner will then position the patient's head in the head stabilizer 10, 118, 146, 162 such that, in the final orientation, the connector plate 32 is disposed proximate to the patient's mouth (e.g., aligned approximately with the patient's mouth). The practitioner will then examine, ventilate, or intubate the patient according to standard, accepted procedures. The practitioner may also secure one or more tubes (e.g., ventilation tubes) to the patient's face using tape, as required or as desired. During examination, ventilation, or intubation, it is contemplated that the practitioner will insert one or more tubes into the patient's mouth and/or nose.

It is contemplated that the medical device will be pre-assembled with a suitable adapter 52 for connection to the connector plate 32. Accordingly, after the patient is intubated, the connector plate 32 and associated arms 28, 30, 164, 166 are attached to the head stabilizer 10, 118, 146, 162.

As should be apparent from the foregoing, the locking portions 48, 50, 172, 174 of the arms 28, 30, 164, 166 are inserted into the channels 102 defined by the locking brackets 24, 26 until the connector plate 32 is at approximately the same height of the adapter 52 disposed on the medical device. Naturally, the adapter 52 on the medical device also may be adjusted in its position, as required or as desired.

As noted above, the slot 86 faces the top edge 20 of the head cradle 12. With this orientation, the slot 86 also is pointed in the same direction as the top of the patient's head.

Once the slot 86 in the connector plate 32 and adapter 52 are approximately aligned with one another, the adapter 52 may be inserted into the slot 86 and rotated (as discussed above) so that the adapter 52 becomes removably attached to the connector plate 32.

At this point, the practitioner may secure the connector plate 32 in its position by closing the levers 36, 38. In so doing, the locking portions 48, 50 of the levers 36, 38 press the locking portions 48, 50, 172, 174 of the arms 28, 30, 164, 166 such that the teeth 64, 66 engage one another, thereby securing the position of the connector plate 32. As should be apparent to those skilled in the art, the exact orientation of the connector plate 32 will depend upon the preferences of the practitioner (among other variables).

With the levers 36, 38 secured, the medical device is anticipated to be fixed in a suitable position to permit the practitioner to complete the procedure on the patient.

As should be apparent from the foregoing, the head stabilizer 10, 118, 146, 162 of the present invention immobilizes the patient's head and, in selected cases, an endotracheal tube, thereby reducing the risk of trauma to the lungs and trachea. The head stabilizer 10, 118, 146, 162 also immobilizes the patient's head and endotracheal tube allowing the physician to move implements such as a bronchoscope without possibility of extubation of the patient. Finally, the head stabilizer 10, 118, 146, 162 immobilizes the patient's head and endotracheal tube allowing the practitioner to position a bronchoscope at a certain level in regard to the endotracheal tube and to be assured at the fact that the depth of the endotracheal tube itself will not change due to movement. Still further aspects of the present invention should be apparent to those skilled in the art.

As should be apparent from the foregoing, the head stabilizer 10, 118, 146, 162 is contemplated to be positioned such that the 14, 16, 122, 124 are adjacent to the sides of a person's head. As such, the connector plate 32 may be positioned above, but near to, the person's nose and mouth. This permits medical instruments to be inserted through the person's nose and mouth, as required or desired for a particular medical procedure.

It is noted that one aspect of the present invention permits medical devices to be connected to the connector plate 32 of the head stabilizer 10, 118, 146, 162 where the medical device is not separately connected to the patient's head. Such a construction may be appropriate in circumstances where the patient has burns on his or her head. In such a circumstance, it is not advisable to tape tubes, wires, or medical devices to damaged tissue to avoid further injury. As should be apparent, this construction is not limited to application to burn victims.

It is also noted that the construction of the head stabilizer 10, 118, 146, 162 of the present invention holds the medical device(s) in the connector plate 32 and/or also in the clamp150. As such, the practitioner's hands may be freed from holding the medical device(s), thereby permitting the practitioner to focus on other activities. This is known more colloquially as a "hands-free" operation. This also helps to reduce the number of persons that may be required to administer a particular medical procedure.

As should also be apparent from the foregoing, the construction of the head stabilizer 10, 118, 146, 162 of the present invention facilitates insertion of medical device(s) into a person's nose and or mouth. However, the head stabilizer 10, 118, 146, 162 may be employed to assist with other medical procedures that do not require oral or nasal insertion, as should be apparent to those skilled in the art.

It is noted that the head stabilizer 10, 118, 146, 162 of the present invention is contemplated to keep medical device(s) stable with respect to the patient. In other words, the head stabilizer 10, 118, 146, 162 is contemplated to stabilize medical device(s) with minimal intervention or manipulation by practitioners or other persons.

As noted above, the present invention is not intended to be limited solely to the embodiments described herein. Those skilled in the art should appreciate certain variations and equivalents of the embodiments described herein. Those variations and equivalents are intended to fall within the scope of the present invention as in appended claims.

## Claims

1. An apparatus for immobilizing a patient's head (10) and suitable for securing a medical device, comprising:
a bottom plate (18);
a first side plate (14) connected to a first side of the bottom plate (18) and extending upwardly from the bottom plate (18);
a second side plate (16) connected to a second side of the bottom plate (18), opposite to the first side, extending upwardly from the bottom plate (18);
a first arm (28) extending from the first plate (14) toward the second plate (16);
a second arm (30) extending from the second plate (16) toward the first plate (14);
a connector plate (32) connected between the first arm (28) and the second arm (30);
an attachment (52) provided on the connector plate (32), wherein the attachment (52) is adapted to secure a first portion of the medical device thereto;
a first locking bracket (24) attached to the first side plate (14) on an exterior surface thereof, wherein the first locking bracket (24) defines a first channel (102) between the first locking bracket (24) and the first side plate (14) adapted to receive a first locking portion (48) of the first arm (28) therein;
a second locking bracket (26) attached to the second side plate (16) on an exterior surface thereof, wherein the second locking bracket (26) defines a second channel (102) between the second locking bracket (26) and the second side plate (16) adapted to receive a second locking portion (50) of the second arm (30) therein;
a first lever (36) disposed on the first locking bracket (24) to secure the first locking portion (48); and
a second lever (38) disposed on the second locking bracket (26) to secure the second locking portion (50).

2. The apparatus (10) of claim 1, wherein the bottom plate (18), the first side plate (14), and the second side plate (16) are substantially planar.

3. The apparatus (10) of claim 1, wherein the bottom plate (18), the first side plate (14), and the second side plate (16) are integrally formed.

4. The apparatus (10) of claim 1, further comprising:
a first tab (126, 128) along a bottom edge of the first side plate (122);
a first slot (130, 132) along a first side edge of the bottom plate (120) to accommodate the first tab (126, 128) therein;
a second tab (126, 128) along a bottom edge of the second side plate (124);
a second slot (130, 132) along a second side edge of the bottom plate (120) to accommodate the second tab (126, 128) therein, the second side edge being opposite to the first side edge;
a first clamp (134) disposed on the bottom plate (120) adjacent to the first side edge to secure the first side plate (122) to the bottom plate (120); and
a second clamp (136) disposed on the bottom plate (120) adjacent to the second side edge to secure the second side plate (124) to the bottom plate (120).

5. The apparatus (10) of claim 4, wherein each clamp (134, 136) comprises:
a retaining portion (138); and
a lever (140) attached to the retaining portion (138),
wherein actuation of the lever (140) causes the retaining portion (138) to engage a retaining slot (142, 144) in each side plate (122, 124).

6. The apparatus (10) of claim 5, wherein the retaining portion (138) is a semi-circular plate (138) pivotally mounted to the bottom plate (120) and the lever (140) is attached to the semi-circular plate (138) such that rotation of the lever (140) causes the semi-circular plate (138) to engage the retaining slot (142, 144).

7. The apparatus of claim 1, further comprising:
a first set of locking teeth (64) disposed on the first locking portion (48);
a first set of side teeth (66) disposed on the first side plate (14) in an orientation providing an interference fit with the first set of locking teeth (64);
a second set of locking teeth (64) disposed on the second locking portion (50); and
a second set of side teeth (66) disposed on the second side plate (16) in an orientation providing an interference fit with the second set of locking teeth (64).

8. The apparatus (10) of claim 7, wherein:
the first lever (36) is L-shaped, defining a first gripping end (44) that presses the first set of locking teeth (64) into engagement with the first set of side teeth (66); and
the second lever (38) is L-shaped, defining a second gripping end (46) that presses the second set of locking teeth (64) into engagement with the second set of side teeth (66).

9. The apparatus (10) of claim 1, wherein the connector plate (32) comprises:
a slot (86) adapted to receive a portion of the medical device therein; and
a lock (88) adapted to secure the portion of the medical device in the slot (86).

10. The apparatus (10) of claim 9, further comprising:
an adapter (52) attachable to the medical device, the adapter (52) being constructed for engagement with the lock (88).

11. The apparatus (10) of claim 10, wherein:
the lock (88) comprises a circular void in the connector plate (32) with a diameter greater than a width of the slot (86); and
the adapter (52) comprises a cylindrical wall (112) with two lateral extensions (114, 116) extending outwardly therefrom and adapted to be inserted into the slot (86) and to subsequently become disposed in the circular void by rotation of the adapter (52) so as to prevent dislodgement of the adapter (52) from the connector plate (32).

12. The apparatus (10) of claim 1, further comprising:
a flexible arm (148); and
a clamp (150) disposed on the flexible arm (148),
wherein the clamp (150) is adapted to releasably engage a second portion of the medical device, different from the first portion, thereby providing additional stability for the medical device.

13. The apparatus (10) of claim 1, wherein:
the first arm (164) includes a first upper arm portion (168) and a first lower arm portion (172), the first upper arm portion (168) being pivotally adjustable with respect to the first lower arm portion (172); and
the second arm (166) includes a second upper arm portion (170) and a second lower arm portion (174), the second upper arm portion (170) being pivotally adjustable with respect to the second lower arm portion (174).

14. The apparatus (10) of claim 1, wherein the apparatus is adapted to secure an endotracheal tube.

## Patentansprüche

1. Gerät zum Stillstellen des Kopfs (10) eines Patienten und geeignet zum Befestigen einer medizinischen Vorrichtung, das Folgendes umfasst:
eine Grundplatte (18);
eine erste Seitenplatte (14), die mit einer ersten Seite der Grundplatte (18) verbunden ist und sich von der Grundplatte (18) aufwärts erstreckt;
eine zweite Seitenplatte (16), die mit einer zweiten Seite der Grundplatte (18), die der ersten Seite entgegengesetzt ist, verbunden ist, die sich von der Grundplatte (18) aufwärts erstreckt;
einen ersten Arm (28), der sich von der ersten Platte (14) zu der zweiten Platte (16) erstreckt;
einen zweiten Arm (30), der sich von der zweiten Platte (16) zu der ersten Platte (14) erstreckt;
eine Verbindungsplatte (32), die zwischen dem ersten Arm (28) und dem zweiten Arm (30) verbunden ist;
eine Befestigung (52), die auf der Verbindungsplatte (32) vorgesehen ist, wobei die Befestigung (52) angepasst ist, um einen ersten Abschnitt der medizinischen Vorrichtung daran zu befestigen;
eine erste Verriegelungsklammer (24), die an der ersten Seitenplatte (14) an einer äußeren Oberfläche angebracht ist, wobei die erste Verriegelungsklammer (24) einen ersten Kanal (102) zwischen der ersten Verriegelungsklammer (24) und der ersten Seitenplatte (14) definiert, der angepasst ist, um einen ersten Verriegelungsabschnitt (48) des ersten Arms (28) darin aufzunehmen;
eine zweite Verriegelungsklammer (26), die an der zweiten Seitenplatte (16) an einer äußeren Oberfläche angebracht ist, wobei die zweite Verriegelungsklammer (26) einen zweiten Kanal (102) zwischen der zweiten Verriegelungsklammer (26) und der zweiten Seitenplatte (16) definiert, der angepasst ist, um einen zweiten Verriegelungsabschnitt (50) des zweiten Arms (30) darin aufzunehmen;
einen ersten Hebel (36), der auf der ersten Verriegelungsklammer (24) angeordnet ist, um den ersten Verriegelungsabschnitt (48) zu befestigen; und
einen zweiten Hebel (38), der auf der zweiten Verriegelungsklammer (26) angeordnet ist, um den zweiten Verriegelungsabschnitt (50) zu befestigen.

2. Gerät (10) nach Anspruch 1, wobei die Grundplatte (18), die erste Seitenplatte (14) und die zweite Seitenplatte (16) im Wesentlichen flach sind.

3. Gerät (10) nach Anspruch 1, wobei die Grundplatte (18), die erste Seitenplatte (14) und die zweite Seitenplatte (16) aus einem Teil ausgebildet sind.

4. Gerät (10) nach Anspruch 1, das ferner Folgendes umfasst:
eine erste Lasche (126, 128) entlang einer Unterkante der ersten Seitenplatte (122);
einen ersten Schlitz (130, 132) entlang einer ersten Seitenkante der Grundplatte (120), um die erste Lasche (126, 128) darin aufzunehmen;
eine zweite Lasche (126, 128) entlang einer Unterkante der zweiten Seitenplatte (124);
einen zweiten Schlitz (130, 132) entlang einer zweiten Seitenkante der Grundplatte (120), um die zweite Lasche (126, 128) darin aufzunehmen, wobei die zweite Seitenkante der ersten Seitenkante entgegengesetzt ist;
eine erste Klemme (134), die auf der Grundplatte (120) benachbart zu der ersten Seitenkante angeordnet ist, um die erste Seitenplatte (122) an der Grundplatte (120) zu befestigen; und
eine zweite Klemme (136), die auf der Grundplatte (120) benachbart zu der zweiten Seitenkante angeordnet ist, um die zweite Seitenplatte (124) an der Grundplatte (120) zu befestigen.

5. Gerät (10) nach Anspruch 4, wobei jede Klemme (134, 136) Folgendes umfasst:
einen Halteabschnitt (138); und
einen Hebel (140), der an dem Halteabschnitt (138) angebracht ist,
wobei das Betätigen des Hebels (140) den Halteabschnitt (138) veranlasst, in einen Halteschlitz (142, 144) in jeder Seitenplatte (122, 124) einzugreifen.

6. Gerät (10) nach Anspruch 5, wobei der Halterabschnitt (138) eine halbkreisförmige Platte (138) ist, die schwenkend an der Grundplatte (120) montiert ist, und der Hebel (140) an der halbkreisförmigen Platte (138) derart angebracht ist, dass die Drehung des Hebels (140) die halbkreisförmige Platte (138) veranlasst, in den Halteschlitz (142, 144) einzugreifen.

7. Gerät nach Anspruch 1, das ferner Folgendes umfasst:
einen ersten Satz von Verriegelungszähnen (64), der auf dem ersten Verriegelungsabschnitt (48) angeordnet ist;
einen ersten Satz von Seitenzähnen (66), der auf der ersten Seitenplatte (14) in einer Ausrichtung angeordnet ist, die eine Presspassung mit dem ersten Satz von Verriegelungszähnen (64) bereitstellt;
einen zweiten Satz von Verriegelungszähnen (64), der auf dem zweiten Verriegelungsabschnitt (50) angeordnet ist; und
einen zweiten Satz von Seitenzähnen (66), der auf der zweiten Seitenplatte (16) in einer Ausrichtung angeordnet ist, die eine Presspassung mit dem zweiten Satz von Verriegelungszähnen (64) bereitstellt.

8. Gerät (10) nach Anspruch 7, wobei:
der erste Hebel (36) L-förmig ist, indem er ein erstes Greifende (44) definiert, das den ersten Satz von Verriegelungszähnen (64) in Eingriff mit dem ersten Satz von Seitenzähnen (66) drückt; und
der zweite Hebel (38) L-förmig ist, indem er ein zweites Greifende (46) definiert, das den zweiten Satz von Verriegelungszähnen (64) in Eingriff mit dem zweiten Satz von Seitenzähnen (66) drückt.

9. Gerät (10) nach Anspruch 1, wobei die Verbindungsplatte (32) Folgendes umfasst:
einen Schlitz (86), der angepasst ist, um einen Abschnitt der medizinischen Vorrichtung in sich aufzunehmen; und
einen Verschluss (88), der angepasst ist, um den Abschnitt der medizinischen Vorrichtung in dem Schlitz (86) zu befestigen.

10. Gerät (10) nach Anspruch 9, das ferner Folgendes umfasst:
einen Adapter (52), der an der medizinischen Vorrichtung angebracht werden kann, wobei der Adapter (52) zum Eingreifen mit dem Verschluss (88) ausgelegt ist.

11. Gerät (10) nach Anspruch 10, wobei:
der Verschluss (88) einen kreisförmigen Leerraum in der Verbindungsplatte (32) mit einem Durchmesser, der größer ist als die Breite des Schlitzes (86), umfasst; und
der Adapter (52) eine zylindrische Wand (112) mit zwei seitlichen Erweiterungen (114, 116), die sich davon auswärts erstrecken und angepasst sind, um in den Schlitz (86) eingefügt zu werden und anschließend in dem kreisförmigen Hohlraum durch Drehung des Adapters (52) angeordnet zu werden, um einem Austreten des Adapters (52) aus der Verbindungsplatte (32) vorzubeugen.

12. Gerät (10) nach Anspruch 1, das ferner Folgendes umfasst:
einen biegsamen Arm (148); und
eine Klemme (150), die auf dem biegsamen Arm (148) angeordnet ist,
wobei die Klemme (150) angepasst ist, um freigebbar einen zweiten Abschnitt der medizinischen Vorrichtung, der von dem ersten Abschnitt unterschiedlich ist, einzurücken, wodurch zusätzliche Stabilität für die medizinische Vorrichtung bereitgestellt ist.

13. Gerät (10) nach Anspruch 1, wobei:
der erste Arm (164) einen ersten oberen Armabschnitt (168) und einen ersten unteren Armabschnitt (172) aufweist, wobei der erste obere Armabschnitt (168) schwenkbar in Bezug zu dem ersten unteren Armabschnitt (172) einstellbar ist; und
der zweite Arm (166) einen zweiten oberen Armabschnitt (170) und einen zweiten unteren Armabschnitt (174) aufweist, wobei der zweite obere Armabschnitt (170) schwenkbar in Bezug zu dem zweiten unteren Armabschnitt (174) einstellbar ist.

14. Gerät (10) nach Anspruch 1, wobei das Gerät angepasst ist, um einen endotrachealen Tubus zu befestigen.

## Revendications

1. Appareil pour immobiliser la tête d'un patient (10) et qui convient pour fixer un dispositif médical, comprenant :
une plaque inférieure (18) ;
une première plaque latérale (14) reliée à un premier côté de la plaque inférieure (18) et s'étendant vers le haut depuis la plaque inférieure (18) ;
une deuxième plaque latérale (16) reliée à un deuxième côté de la plaque inférieure (18), opposé au premier côté, s'étendant vers le haut depuis la plaque inférieure (18) ;
un premier bras (28) s'étendant de la première plaque (14) vers la deuxième plaque (16) ;
un deuxième bras (30) s'étendant de la deuxième plaque (16) vers la première plaque (14) ;
une plaque de connexion (32) reliée entre le premier bras (28) et le deuxième bras (30) ;
un accessoire (52) fourni sur la plaque de connexion (32), dans lequel l'accessoire (52) est adapté pour fixer une première portion du dispositif médical sur celui-ci ;
un premier support de verrouillage (24) attaché à la première plaque latérale (14) sur une surface extérieure de celle-ci, dans lequel le premier support de verrouillage (24) définit un premier canal (102) entre le premier support de verrouillage (24) et la première plaque latérale (14) adapté pour recevoir une première portion de verrouillage (48) du premier bras (28) à l'intérieur de celui-ci ;
un deuxième support de verrouillage (26) attaché à la deuxième plaque latérale (16) sur une surface extérieure de celle-ci, dans lequel le deuxième support de verrouillage (26) définit un deuxième canal (102) entre le deuxième support de verrouillage (26) et la deuxième plaque latérale (16) adapté pour recevoir une deuxième portion de verrouillage (50) du deuxième bras (30) à l'intérieur de celui-ci ;
un premier levier (36) disposé sur le premier support de verrouillage (24) pour fixer la première portion de verrouillage (48) ; et
un deuxième levier (38) disposé sur le deuxième support de verrouillage (26) pour fixer la deuxième portion de verrouillage (50).

2. Appareil (10) selon la revendication 1, dans lequel la plaque inférieure (18), la première plaque latérale (14), et la deuxième plaque latérale (16) sont sensiblement planes.

3. Appareil (10) selon la revendication 1, dans lequel la plaque inférieure (18), la première plaque latérale (14), et la deuxième plaque latérale (16) sont formées d'un seul tenant.

4. Appareil (10) selon la revendication 1, comprenant en outre :
une première patte (126, 128) le long d'un bord inférieur de la première plaque latérale (122) ;
une première fente (130, 132) le long d'un premier bord latéral de la plaque inférieure (120) pour loger la première patte (126, 128) à l'intérieur de celle-ci ;
une deuxième patte (126, 128) le long d'un bord inférieur de la deuxième plaque latérale (124) ;
une deuxième fente (130, 132) le long d'un deuxième bord latéral de la plaque inférieure (120) pour loger la deuxième patte (126, 128) à l'intérieur de celle-ci, le deuxième bord latéral étant opposé au premier bord latéral ;
une première pince (134) disposée sur la plaque inférieure (120) adjacente au premier bord latéral pour fixer la première plaque latérale (122) à la plaque inférieure (120) ; et
une deuxième pince (136) disposée sur la plaque inférieure (120) adjacente au deuxième bord latéral pour fixer la deuxième plaque latérale (124) à la plaque inférieure (120).

5. Appareil (10) selon la revendication 4, dans lequel chaque pince (134, 136) comprend :
une portion de retenue (138) ; et
un levier (140) attaché à la portion de retenue (138),
dans lequel l'actionnement du levier (140) amène la portion de retenue (138) à entrer en prise avec une fente de retenue (142, 144) dans chaque plaque latérale (122, 124).

6. Appareil (10) selon la revendication 5, dans lequel la portion de retenue (138) est une plaque semi-circulaire (138) montée de manière pivotante à la plaque inférieure (120) et le levier (140) est attaché à la plaque semi-circulaire (138) de façon à ce que la rotation du levier (140) amène la plaque semi-circulaire (138) à entrer en prise avec la fente de retenue (142, 144).

7. Appareil selon la revendication 1, comprenant en outre :
un premier jeu de dents de verrouillage (64) disposé sur la première portion de verrouillage (48) ;
un premier jeu de dents latérales (66) disposé sur la première plaque latérale (14) dans une orientation fournissant un ajustement serré avec le premier jeu de dents de verrouillage (64) ;
un deuxième jeu de dents de verrouillage (64) disposé sur la deuxième portion de verrouillage (50) ; et
un deuxième jeu de dents latérales (66) disposé sur la deuxième plaque latérale (16) dans une orientation fournissant un ajustement serré avec le deuxième jeu de dents de verrouillage (64).

8. Appareil (10) selon la revendication 7, dans lequel :
le premier levier (36) est en forme de L, définissant une première extrémité de préhension (44) qui presse le premier jeu de dents de verrouillage (64) en prise avec le premier jeu de dents latérales (66) ; et
le deuxième levier (38) est en forme de L, définissant une deuxième extrémité de préhension (46) qui presse le deuxième jeu de dents de verrouillage (64) en prise avec le deuxième jeu de dents latérales (66).

9. Appareil (10) selon la revendication 1, dans lequel la plaque de connexion (32) comprend :
une fente (86) adaptée pour recevoir une portion du dispositif médical à l'intérieur de celle-ci ; et
un verrou (88) adapté pour fixer la portion du dispositif médical dans la fente (86).

10. Appareil (10) selon la revendication 9, comprenant en outre :
un adaptateur (52) pouvant être attaché au dispositif médical, l'adaptateur (52) étant construit pour entrer en prise avec le verrou (88).

11. Appareil (10) selon la revendication 10, dans lequel :
le verrou (88) comprend un évidement circulaire dans la plaque de connexion (32) d'un diamètre supérieur à une largeur de la fente (86) ; et
l'adaptateur (52) comprend une paroi cylindrique (112) avec deux prolongements latéraux (114, 116) s'étendant vers l'extérieur de celle-ci et adaptée pour être insérée dans la fente (86) et pour être ensuite disposée dans l'évidement circulaire par rotation de l'adaptateur (52) de façon à empêcher un délogement de l'adaptateur (52) de la plaque de connexion (32).

12. Appareil (10) selon la revendication 1, comprenant en outre :
un bras flexible (148) ; et
une pince (150) disposée sur le bras flexible (148),
dans lequel la pince (150) est adaptée pour entrer en prise de manière libérable avec une deuxième portion du dispositif médical, différente de la première portion, fournissant ainsi une stabilité supplémentaire au dispositif médical.

13. Appareil (10) selon la revendication 1, dans lequel :
le premier bras (164) comprend une première portion de bras supérieure (168) et une première portion de bras inférieure (172), la première portion de bras supérieure (168) étant réglage de manière pivotante par rapport à la première portion de bras inférieure (172) ; et
le deuxième bras (166) comprend une deuxième portion de bras supérieure (170) et une deuxième portion de bras inférieure (174), la deuxième portion de bras supérieure (170) étant réglage de manière pivotante par rapport à la deuxième portion de bras inférieure (174).

14. Appareil (10) selon la revendication 1, dans lequel l'appareil est adapté pour fixer un tube endotrachéal.
